Europäisches Patentamt

European Patent Office

Office européen des brevets

(11) Publication number: **0 057 066**
**B1**

(12) EUROPEAN PATENT SPECIFICATION

(45) Date of publication of patent specification: **13.03.85**

(51) Int. Cl.⁴: **C 07 D 301/10, C 07 D 303/04**
**// B01J23/66**

(21) Application number: **82300192.0**

(22) Date of filing: **14.01.82**

(54) **Process for the production of ethylene oxide.**

(30) Priority: **23.01.81 GB 8102119**

(43) Date of publication of application:
**04.08.82 Bulletin 82/31**

(45) Publication of the grant of the patent:
**13.03.85 Bulletin 85/11**

(84) Designated Contracting States:
**BE DE FR GB IT NL SE**

(56) References cited:
**GB-A-1 213 483**
**GB-A-1 314 613**
**GB-A-1 382 099**
**GB-A-1 512 625**
**GB-A-1 563 051**
**GB-A-2 042 362**
**GB-A-2 045 636**

(73) Proprietor: **IMPERIAL CHEMICAL INDUSTRIES PLC**
**Imperial Chemical House Millbank**
**London SW1P 3JF (GB)**

(72) Inventor: **Clayton, Richard William**
**18 Goathland Grove**
**Guisborough Cleveland (GB)**
Inventor: **Hayden, Percy**
**2 Hawthorne Drive Hutton Meadows**
**Guisborough Cleveland (GB)**
Inventor: **Johnson, David William**
**15 Princess Mount**
**Knaresbrough North Yorkshire (GB)**

(74) Representative: **Locke, Timothy John et al**
**Imperial Chemical Industries PLC Legal**
**Department: Patents Po Box 6**
**Welwyn Garden City Herts, AL7 1HD (GB)**

Courier Press, Leamington Spa, England.

## Description

This invention relates to a process for the production of ethylene oxide.

Ethylene oxide is conventionally produced by contacting ethylene and oxygen with a supported silver catalyst in the presence of a chlorine containing reaction inhibitor or modifier which is present in the gas phase. The inhibitor or modifier is normally ethylene dichloride and/or vinyl chloride and its function is to increase the selectivity of the reaction (i.e. to increase number of moles of ethylene oxide produced per hundred moles of ethylene consumed). This reduces the activity of the catalyst; the reduction in activity does however reduce the danger of "hot spots" in catalyst beds.

In GB—A—1,512,625 a silver catalyst for use in the oxidation of ethylene to ethylene oxide is disclosed which contains potassium, rubidium and/or cesium coincidentally deposited with the silver.

In GB—A—2,045,636 a process is described in which a silver containing catalyst is prepared by impregnating a support with a decomposable silver compound and decomposing it to leave silver and residues on a support, removing the residues and post impregnating the catalyst with a solution of one or more alkali metals selected from cesium, rubidium and potassium. In one of the Examples (Example 2) a catalyst containing 50 parts per million of cesium was tested for the oxidation of ethylene to ethylene oxide in a gas stream containing 0.2% ethane and 0.25 ppm ethylene dichloride.

Processes for the production of ethylene oxide using promoted silver catalysts are known from GB—A—2,042,362 and GB—A—1,563,051; processes using certain ethane/inhibitor ratios are described in GBT—A—1,382,099 and GB—A—1,273,483.

We have found that in certain attractive catalysts chlorine is permanently absorbed from the modifier on to the catalyst bed and this gives rise to accelerated decay of the catalysts performance. We have devised conditions under which absorption of this type may be reduced to an acceptable level.

The invention comprises a process of producing ethylene oxide by contacting ethylene with oxygen optionally in the presence of diluent gases and in the presence of a chlorine containing reaction inhibitor and a silver containing catalyst which is promoted with potassium, rubidium and/or cesium characterised in that ethane is present in a ratio of at least 5,000, preferably at least 8,000, and more preferably at least 10,000, to 100,000 moles per mole of reaction inhibitor expressed as its vinyl chloride equivalent, the concentration of the reaction inhibitor expressed as its vinyl chloride equivalent is 0.2 to 10 parts per million by volume and that the potassium, rubidium and/or cesium content is at least $5 \times 10^{-6}$ and preferably more than $8 \times 10^{-6}$ and suitably at most $25 \times 10^{-6}$ gram atoms per gram of catalyst in a form which is extractable by contact with dilute nitric acid.

The promoter is preferably cesium.

Chlorine containing reaction inhibitors are of different effectiveness according to their chemical structure. We have found that 0.5 moles of ethylene dichloride, 2 moles of ethyl chloride and 12 moles of methyl chloride are equivalent to one mole of vinyl chloride. The effectiveness of other chlorine containing compounds may readily be determined empirically. By the vinyl chloride equivalent of the reaction inhibitor we mean the amount of the inhibitor necessary to produce the same inhibition as is produced by one mole of vinyl chloride.

The process is suitably carried out in a fixed bed of catalyst which may be contained in the tubes of a multi-tubular reactor. The chemical composition of the reaction inhibitor tends to change as it passes through the catalyst bed at least in part because of lay-down and removal of chlorine from the catalyst and consequently the vinyl chloride equivalent of the inhibitor may vary along the bed. If gases containing reaction inhibitor are recycled to the reactor the composition of the reaction inhibitor will tend to approach an equilibrium but if newly fed inhibitor does not correspond to that equilibrium the vinyl chloride equivalent of inhibitor may still vary along the bed. For this and other reasons it may be desirable to have catalyst with different promoter contents at the inlet and outlet ends of the bed as high levels of alkali metal tend to lead to increased permanent absorption of chlorine into the catalyst. If the reaction inhibitor is methyl chloride for example a higher alkali metal content will be desirable at the inlet end of the bed than at the outlet end of the bed because methyl chloride is comparatively ineffective as a chlorine donator, whereas if ethylene dichloride or vinyl chloride are fed as the reaction inhibitor it may be desirable to have a reduced alkali metal content at the inlet to the bed. The amounts of reaction inhibitor and ethane specified in this invention are those fed to the inlet of the catalyst bed however.

The catalyst comprises silver, suitably supported on a porous heat resisting support which may have a specific surface area in the range 0.05 to 10 m²/g preferably 1 to 5 m²/g and more preferably 1.5 to 5 m²/g as measured by the Brunauer Emmett and Teller method, the catalyst comprising the promoting amount of potassium, rubidium and/or cesium, in excess of any present in the support as impurities or cements.

The silver may be introduced to a preformed porous heat resisting support as a suspension of silver or silver oxide in a liquid medium for example water or by impregnation of the support with a solution of a silver compound, for example silver nitrate, which can be reduced to silver metal. If necessary a reducing agent, for example hydrogen, may be employed but a heat treatment may however suffice to decompose the silver compound to silver. Suitably the impregnating solution contains a reducing agent which may be for example an anion, for example a formate, acetate, propionate, lactate, oxalate or tartarate ion, of a silver compound in the solution. The reducing agent may be for example an aldehyde, for example formaldehyde or acetaldehyde or an alcohol preferably having 1 to 4 carbon atoms for example methanol or ethanol.

2

O 057 066

The solution may be a solution in water and/or an organic solvent, for example an aliphatic alcohol preferably having 1 to 4 carbon atoms; a polyhydric alcohol for example ethylene glycol or glycerol; a ketone for example acetone; an ether for example dioxan or tetrahydrofuran; a carboxylic acid for example acetic acid or preferably molten lactic acid which is preferably used in the presence of water and suitably in the presence of an oxidising agent for example $H_2O_2$; or an ester for example ethyl acetate or a nitrogen containing base for example pyridine or formamide. An organic solvent may function as a reducing agent and/or complexing agent for the silver also.

If the silver is introduced by impregnating a support with a solution of a decomposable silver compound it is preferred that ammonia and/or a nitrogen containing base should be present. The nitrogen containing base suitably acts as a ligand maintaining the silver in solution; for example it may be pyridine, acetonitrile, an amine, especially a primary or secondary amine having 1—6 carbon atoms, for example isopropylamine. Other suitable nitrogen-containing bases include ammonia, hydroxylamine and alkanolamines for example ethanolamine, alkylene diamines having from 2—4 carbon atoms or polyamines containing at least three carbon atoms for example diethylenetriamine or amino ethers with at least one ether linkage and at least one primary or secondary amino group e.g. morpholine or amides for example formamide or dimethyl formamide. The nitrogen containing bases may be used alone or in admixture. They also act as reducing agents for the silver compound. Suitably the nitrogen containing base or bases are used together with water.

Very suitably the solution comprises silver nitrate and a primary or secondary diamine having 1 to 6 carbon atoms for example isopropylamine and water.

Alternatively the solution may be a neutral or acid solution for example it may be a solution of a silver carboxylate especially a formate, acetate, propionate, oxalate, citrate, tartarate or preferably lactate or for example a solution of silver nitrate.

The solutions preferably contain 3—50% of silver by weight.

Impregnation may be carried out in a single stage or if desired may be repeated one or more times. By this means higher silver contents of the catalyst may be achieved.

The silver compound may be decomposed for example in an atmosphere of air or preferably nitrogen.

The silver compound may generally be partially or completely reduced to silver by heating in the range 100 to 350°C, for example for a period of 5 mins to 10 hours; alternatively reduction may be carried out during the oxidation of the olefine to the olefine oxide.

The catalyst support preferably has an apparent porosity as measured by the mercury absorption method of at least 20%, for example 30—80% preferably 30—65% and more preferably 35—65% and mean pore diameters of 0.1 to 20 microns preferably 0.3 to 4 microns as measured by the mercury porosimetry method. The pore size distribution of the support may be bimodal, in which case the smaller pores preferably account for at least 70% of the total pore volume and have a mean pore diameter preferably in the range of 0.1 and preferably 0.3 to 4 microns, and the larger pores preferably have a mean pore diameter in the range 25 to 500 microns.

Most of the silver content of the catalyst is preferably present in the form of discrete particles adhering to the support having equivalent diameters of less than 10,000Å preferably in the range 20—10,000Å and more preferably 40—8,000Å for example 100—5,000Å. By equivalent diameter is meant the diameter of a hemisphere of the same silver content as the particle.

Preferably at least 80% of the silver is present as particles having equivalent diameters in the aforesaid range, the quantity of silver being judged in terms of the number of particles falling in that range. The silver is believed to be present largely as metallic silver. The dimensions of the silver particles may be determined by scanning electron microscopy.

The support may be an alumina, silicon carbide, silica, zirconia, titania or silica/alumina support, but it is preferably composed of an aggregate of alpha-alumina particles which may be fused together or cemented together with, for example silica or baryta.

The catalyst preferably comprises 3 to 50% and more preferably 3 to 30% for example 6 to 28% by weight of silver.

The potassium, rubidium or cesium may be introduced to the support before, during or after the introduction of the silver compound. Preferably it is introduced to a support on which the silver is substantially present in metallic form. It is suitably introduced in solution in water and/or organic solvents. If it is desired to impregnate a catalyst which has already been used in the oxidation of an alkene to an alkylene oxide and has lost performance, this may be carried out also.

The potassium, rubidium and/or cesium are preferably introduced as salts for example carbonates or carboxylates. Polycarboxylic acids and hydroxyacids are particularly suitable. An example of a particularly suitable acid is oxalic acid.

Partial pressures of ethylene in processes according to the invention may be in the ranges 0.1—30 and preferably 1 to 30 bars. The total pressure may be in the range of from 1 to 100 and preferably 3—100 bars absolute. The molar ratio of oxygen to ethylene may be in the range 0.05 to 100. The partial pressure of oxygen may be in the range 0.01 and preferably 0.1 to 20 bars and preferably 1—10 bars. The oxygen may be supplied for example in the form of air or preferably as commercial oxygen. A diluent for example, helium, nitrogen, argon, carbon dioxide and/or a lower paraffin other than ethane which is preferably methane may be present in proportions of 10—80% and preferably 40—70% by volume in total. The

3

amount of ethane present is suitably in the range 0.2 to 10% and preferably 0.3 to 5% by volume of the gases fed to the reaction. Suitably the diluent comprisesd methane as aforesaid together with, for example 2,000 to 100,000 and preferably 5,000 to 80,000 parts per million by total volume of ethane, preferably together with small amount, for example 10 to 10,000 parts per million by total volume of $C_3$ to $C_6$ alkanes, cycloalkanes or alkenes preferably propylene, cyclopropane, isobutene or isobutane. It is necessary to operate using gas compositions which are outside the explosive limits.

The temperature is suitably in the range 170 to 320°C, preferably 180 to 300°C and more preferably in the range 190 to 270°C. Contact times should be sufficient to convert 0.5—70%, for example 2 to 20 and preferably 5—20% of the ethylene and unconverted ethylene is, after separation of the product, suitably recycled, optionally in the presence of unconverted oxygen where appropriate and suitably after removal of $CO_2$.

The chlorine-containing reaction modifier may be of known type. It is preferred that it should be a $C_1$ to $C_{10}$ compound also containing hydrogen. It may be for example 1,1 or preferably 1,2-dichlorethane but it is preferred to use vinyl chloride or more preferably methyl or ethyl chloride. Chlorinated aromatic compounds, for example chlorobenzene, dichlorobenzene and chlorinated toluenes are also suitable. The concentration of the chlorine containing reaction modifier is 0.2 to 10 parts per million by volume expressed as its vinyl chloride equivalent, preferably 0.2 to 3 ppm.

## Example 1

The catalysts described in Examples 2—5 were prepared by the following method:

A solution of silver nitrate/isopropylamine complex in water was prepared by adding isopropylamine (2.2 moles) to an aqueous solution of silver nitrate (1 mole) to produce a solution containing 430 g Ag/l. The solution of the complex was used to impregnate an α-alumina support with a surface area of 2 $m^2$/g, pore volume 0.38 $cm^3$ $g^{-1}$, and mean pore diameter 0.8 μm. After soaking for 15 mins the wet support was drained for 15 mins and then transferred to a pyrolysis unit. Pyrolysis was carried out in a stream of nitrogen for two hours at 90°C, for three hours at 155°C and finally for one and a half hours at 240°C. The silver content of the finished catalyst was approximately 14% by weight.

The cesium promoter was applied by soaking the catalyst for 15 mins in a solution of anhydrous $Cs_2CO_3$ in methanol (0.31 g per 100 ml). The excess solution was decanted and the wet catalyst drained for 15 mins before drying in a stream of nitrogen at 120°C for one hour.

## Example 2

Two catalysts (A and B) containing approximately 1,000 parts per million by weight of cesium were prepared as in Example 1.

The performance of catalysts A and B were determined as follows. 140 grams of catalyst in the form of 2 mm particles was loaded into an 11 mm internal diameter stainless steel reactor. A process gas containing 30% ethylene, 8% oxygen, 4% $CO_2$, vinyl chloride, ethane and the remainder nitrogen was passed over the catalyst at a gas-hourly-space-velocity of 3,760 $hr^{-1}$ and a pressure of 16 bars absolute. The reactor temperature was raised until an oxygen conversion of 40% was achieved. The catalyst selectivity at 40% oxygen conversion and the temperature required to achieve 40% oxygen conversion were recorded over a period of 90 days. The performances achieved using different levels of ethane are displayed in Table 1. The catalyst tested at 2.4% ethane displayed a stable selectivity and only a slow fall in activity. That tested at 0.6% ethane was initially more selective but selectivity and activity decreased rapidly with time. At this lower ethane level, catalyst A is not suitable for longer term operation at the indicated level of vinyl chloride. R indicates the ethane/inhibitor ratio (moles/moles). The processes performed with catalyst A are not claimed.

TABLE 1

| Catalyst | Vinyl Chloride (ppm by volume | Ethane (% by volume) | R | Performance at Day 10 | | Performance at Day 90 | |
|---|---|---|---|---|---|---|---|
| | | | | S40 (%) | T40 (°C) | S40 (%) | T40 (°C) |
| A | 3 | 0.6 | 2000 | 78.4 | 215 | 76.1 | 232 |
| B | 3 | 2.4 | 8000 | 76.3 | 201 | 76.2 | 207 |

On completion of this test the catalyst was discharged in approximately equal sections and the chloride level of each section determined using neutron activation analysis.

The results are presented in Table 2; untested samples of catalyst A and B contained less than 5 ppm chloride.

4

TABLE 2

| Catalyst | Vinyl Chloride (ppm) | Ethane (% by volume) | R | Catalyst chloride level (ppm) Front of ⟶ Back of bed                    bed |
|----------|----------------------|----------------------|------|-----------------------------------------------------------------------|
| A | 3 | 0.6 | 2000 | 4100, 4900, 1200, 490, 96, 69, 72 |
| B | 3 | 2.4 | 8000 | 150, 310, 215, 115, 122, 136 |

Example 3

Two catalysts (C & D) were prepared as described in Example 1 except that the support had a surface area of 2 $m^2g^{-1}$, pore volume 0.3 $cm^3g^{-1}$ and mean diameter 0.6 µm, and, in order to introduce 14% silver by weight silver was added in two successive similar impregnations, the impregnated catalyst being dried at 90°C for 1 hour between impregnations. In both cases the cesium promoter was added to the previously silvered support as a solution of $Cs_2CO_3$ in methanol containing 0.32 and 0.37 g cesium/100 ml solvent respectively. Catalyst C contained 1180 ppm cesium and catalyst D contained 1250 ppm cesium by weight. The performances of catalyst C and D were determined using the same experimental procedure described in Example 2. Samples of catalyst C and D were tested in the presence of various levels of organochloro compounds and with various levels of ethane. The selectivities recorded at 40% oxygen conversion and the temperatures required to achieve 40% oxygen conversion at the end of the 14 days are presented in Table 3 together with the levels of organochloro — compound and ethane used. After each test which lasted about 14 days, samples of catalyst situated at the front, middle and back of the catalyst bed were analysed for chloride level using neutron activation analysis. These data are also shown in Table 3.

The results recorded at the end of the 14 day period show that the catalysts accumulate non uniform levels of chloride at low ratios of ethane to reaction inhibitor. The temperatures are also lower when feeding a high ratio of ethane to vinyl chloride equivalent. These tests were too short to show the selectivity changes seen in Example 2.

The first 2 runs with a ethane/inhibitor ratio of zero and 3000 are not comprised by claim 1.

TABLE 3

| Type of chloro compound | Level of chloro compound | Level of ethane (% by volume) | R | CATALYST C 1250 ppm Cesium | | | | | | CATALYST D 1180 ppm Cesium | | | | |
|---|---|---|---|---|---|---|---|---|---|---|---|---|---|---|
| | | | | S40 % | T40 °C | Chloride level after test (ppm) | | | S40 % | T40 °C | Chloride level after test (ppm) | | | |
| | | | | | | Front | Middle | Back | | | Front | Middle | Back | |
| VC | 3 | 0.6 | 2000* | 77.4 | 225 | 1910 | 950 | 120 | 76.1 | 220 | — | — | — | |
| VC | 2 | 0.6 | 3000* | 77.4 | 220 | 2350 | 1094 | 108 | 76.1 | 213 | — | — | — | |
| VC | 1 | 0.6 | 6000 | 77.0 | 210 | 1140 | 210 | 110 | 75.3 | 211 | 917 | 150 | 90 | |
| VC | 3 | 2.4 | 8000 | 76.0 | 203 | 150 | 120 | 140 | 74.7 | 209 | 1000 | 250 | 110 | |
| VC | 2 | 2.4 | 12000 | 75.5 | 201 | 56 | 80 | 70 | 75.1 | 206 | 170 | 107 | 96 | |
| VC | 1 | 2.4 | 24000 | 75.0 | 198 | 50 | 120 | 130 | 74.6 | 203 | 96 | 86 | 100 | |
| MC | 10 | 0.6 | 7200 | 75.9 | 209 | 164 | 1060 | 880 | — | — | — | — | — | |
| MC | 6 | 0.6 | 12000 | 76.1 | 206 | — | — | — | 76.3 | 211 | 84 | 506 | 443 | |
| MC | 3 | 0.6 | 24000 | 75.1 | 203 | 130 | 250 | 215 | 75.2 | 204 | 96 | 270 | 240 | |

VC means vinyl chloride

MC means methyl chloride

R = ethane/inhibitor ratio

## 0 057 066

### Example 4

Catalyst E to L were prepared following the method described in Example 3. The cesium promoter was added in the form of a solution of $Cs_2 CO_3$ in methanol. The cesium levels present in catalysts E to L were determined by neutron activation analysis. The results are displayed in Table 4. The performances were determined in the following way: 20 grams of catalyst in the form of 1 mm particles were loaded into a stainless steel reactor with an internal diameter of 8 mm. The temperature was raised in the presence of a process gas stream containing 30% ethylene, 7% oxygen with the remainder nitrogen together with ethane and vinyl chloride (VC). The gas hourly space velocity was 4,000 $hr^{-1}$ and the pressure was 225 psig. Different samples of catalysts E to L were tested in the presence of different levels of vinyl chloride and ethane. The levels usd, together with the catalyst selectivity at 40% oxygen conversion and the temperature required to achieve 40% oxygen conversion, are presented in Table 4. These performances were measured after about two weeks on line. In addition, some of these samples were analysed for chloride content using neutron activation analysis. Samples were taken from the front, middle and back of each catalyst bed. These data are also displayed in Table 4. Selectivities are highest and the catalyst chloride levels more uniform at high ratios of ethane to vinyl chloride equivalent.

The first 2 runs refer to a ethane/inhibitor ratio of 2000 and 3000 which is not subject of claim 1.

TABLE 4

| Type of Chloride | Level of Chloride (ppm) | Level of Ethane (%) | R | E * 250 ppm Cs | | F * 350 ppm Cs | | G 660 ppm Cs | | H 850 ppm Cs | | | | |
|---|---|---|---|---|---|---|---|---|---|---|---|---|---|---|
| | | | | S40 % | T40 °C | S40 % | T40 °C | S40 % | T40 °C | S40 % | T40 °C | Chloride level | | |
| | | | | | | | | | | | | Front | Middle | Back |
| VC | 3 | 0.6 | 2000* | — | — | — | — | 75.9 | 207 | 78.3 | 192 | 684 | 206 | 178 |
| VC | 2 | 0.6 | 3000* | — | — | — | — | — | — | 78.2 | 196 | 412 | 170 | 166 |
| VC | 1 | 0.6 | 6000 | 77.3 | 194 | 77.9 | 190 | — | — | 79.1 | 198 | 216 | 143 | 160 |
| VC | 0.5 | 0.6 | 12000 | 77.3 | 201 | 77.8 | 189 | 78.6 | 185 | 78.6 | 183 | — | — | — |
| VC | 2.0 | 2.4 | 12000 | — | — | 77.7 | 194 | 77.8 | 190 | 78.2 | 190 | 164 | 161 | 127 |
| VC | 1.0 | 2.4 | 24000 | — | — | — | — | — | — | 75.8 | 185 | — | — | — |

TABLE 4 (Continued)

| Type of Chloride | Level of Chloride (ppm) | Level of Ethane (%) | R | I 1200 ppm Cs | | | | | | J 1400 ppm Cs | | | | |
|---|---|---|---|---|---|---|---|---|---|---|---|---|---|---|
| | | | | S40 % | T40 °C | Chlorine level | | | S40 % | T40 °C | Chloride level | | |
| | | | | | | Front | Middle | Back | | | Front | Middle | Back |
| VC | 3 | 0.6 | 2000* | 78.9 | 199 | 955 | 224 | 262 | — | — | — | — | — |
| VC | 2 | 0.6 | 3000* | 79.6 | 202 | 890 | 212 | 189 | 79.5 | 207 | 1266 | 168 | 150 |
| VC | 1 | 0.6 | 6000 | 79.7 | 205 | 925 | 147 | 150 | 78.9 | 200 | — | — | — |
| VC | 0.5 | 0.6 | 12000 | 78.5 | 190 | — | — | — | — | — | — | — | — |
| VC | 2.0 | 2.4 | 12000 | 79.9 | 195 | 442 | 179 | 156 | — | — | — | — | — |
| VC | 1.0 | 2.4 | 24000 | 75.8 | 195 | — | — | — | — | — | — | — | — |

TABLE 4 (Continued)

| Type of Chloride | Level of Chloride (ppm) | Level of Ethane (%) | R | K 1700 ppm Cs | | L 2000 ppm Cs | |
|---|---|---|---|---|---|---|---|
| | | | | S40 % | T40 °C | S40 % | T40 °C |
| VC | 3 | 0.6 | 2000* | — | — | — | — |
| VC | 2 | 0.6 | 3000* | — | — | 76.0 | 221 |
| VC | 1 | 0.6 | 6000 | — | — | 78.8 | 213 |
| VC | 0.5 | 0.6 | 12000 | — | — | 76.7 | 194 |
| VC | 2.0 | 2.4 | 12000 | 77.4 | 204 | 76.9 | 200 |
| VC | 1.0 | 2.4 | 24000 | — | — | — | — |

R = ethane/inhibitor ratio

## Example 5

Catalyst M was prepared using the method described in Example 3. The catalyst contained 14% (w/w) silver and 1100 ppm cesium, added as cesium carbonate in methanol.

0.4 g of catalyst M was tested for ethylene oxidation in the presence of a range of chlorocompounds in a stainless steel reactor 2 mm in diameter and 10 cm long. Catalyst M was exposed to a mixture containing 8% oxygen, 3.5% carbon dioxide, 30% ethylene, 0.6% ethane, 58% nitrogen and a low level of chlorocompound, at 225 psig pressure and a GHSV of 3000 $hr^{-1}$. The level of chlorocompound was adjusted until the catalyst gave an activity of 4 moles of ethylene converted per litre of reactor per hour at 40% conversion of oxygen at 200°C. The levels of chlorocompound and selectivities to ethylene oxide are shown in Table 5. The catalyst performance measured at 6 ppm methyl chloride was constant over 50 days.

TABLE 5

| Chlorocompound | Level ppm | R | Selectivity to Ethyleneoxide (%) |
|---|---|---|---|
| Methyl Chloride | 6.0 | 12000 | 77.5 |
| Ethyl Chloride | 1.0 | 12000 | 77.4 |
| Vinyl Chloride | 0.3 | 20000 | 77.2 |

R = ethane/inhibitor ratio.

## Example 6

Catalysts N—R were prepared using the method described in Example 3. They contained 14% (w/w) silver and cesium loadings between 1000 and 1500 ppm added as cesium oxalate in methanol. Catalysts were tested for ethylene oxidation as described in Example 4, but with 6 ppm methyl chloride and 0.6% ethane (R=12000). The performance of these catalysts is presented in Table 6.

10

**0 057 066**

TABLE 6

| Catalyst | Cesium (ppm) | Selectivity at 40% Conversion (%) | Temperature (°C) |
|---|---|---|---|
| N | 1000 | 78.6 | 195 |
| O | 1100 | 78.7 | 190 |
| P | 1200 | 79.1 | 190 |
| Q | 1300 | 79.2 | 188 |
| R | 1500 | 78.5 | 193 |

Example 7

A silver catalyst S was prepared using the method described in Example 3. The catalyst contained 14% by weight of silver and about 800 ppm of Cs. The Cs was added as $Cs_2 CO_3$. 140 g of catalyst S in the form of 2 mm particles was loaded into a stainless steel reactor with an 11 mm internal diameter. A process gas containing 30% ethylene, 8% oxygen, 4% $CO_2$ 6 ppm methyl chloride, 0.6% ethane (R=12000) and the remainder nitrogen was passed over the catalyst at a gas hourly space velocity of 3760 $hr^{-1}$ and at a pressure of 225 psig. The performance of this catalyst was monitored over a period of 28 days. The chloride level on the discharged catalyst was also determined as a function of the position in the bed using neutron activation analysis. The performances and chloride levels obtained are displayed in Table 7.

Catalyst S was then replaced in the reactor with the front 20 g replaced with 20 g of catalyst T. Catalyst T was identical to catalyst S except that it contained 600 ppm Cs. The performance of this combination (20 g catalyst T plus 120 g of catalyst S) was determined over a period of 7 days, using the procedure used to test catalyst S. The results are given in Table 7. Catalysts S and T were then discharged. Catalyst S was then replaced in the reactor with 20 gm of catalyst U as the front end. Catalyst U was identical to catalyst S except that it contained 1600 ppm Cs. The performance of this combination (20 g catalyst U plus 120 g catalyst S) was determined using the same procedure over a period of 7 days. In addition the chloride level was determined as a function of bed position. The results are presented in Table 7.

TABLE 7

| Catalyst | | Methyl Chloride ppm | Selectivity % | Oxygen Conversion % | Temperature | Catalyst Chloride Level (ppm) (Front to back) |
|---|---|---|---|---|---|---|
| Front End | Back & Middle | | | | | |
| S | S | 6 | 77.4 | 30.9 | 198 | 100, 600, 590, 508, 525 |
| T | S | 6 | 76.1 | 28.6 | 198 | — |
| U | S | 6 | 77.1 | 40.1 | 212 | 417, 740, 800, 765, 712 |

Example 8

A catalyst V was prepared as in Example 1 on an α-alumina support of surface area 1.8 $m^2/g$, pore volume 0.28 $cm^3/g$ and mean pore diameter 0.6 μm. The catalyst contained 9.5% siliver w/w. The catalyst was washed with distilled water at 95°C at a liquid hourly space velocity of 5 kg/kg/hr for 8 hours. Cesium was impregnated onto the catalyst from an excess of solution (1 ml solution/g catalyst) containing 0.22 g cesium oxalate/100 ml methanol/water mixture (98:2 v/v). After 16 hours immersion the catalyst was drained and dried at 120°C for 1 hour. The catalyst contained 1000 ppm cesium.

11

Duplicate samples of this catalyst were tested as in Example 2. 100 g of catalyst in the form of 420—1000 µm particles were charged to a stainless steel reactor with an 11 mm internal diameter. A process gas containing 30% ethylene, 8% oxygen, 4% carbon dioxide, 0.6% ethane and organochloride and the remainder nitrogen was passed over the catalyst at a gas hourly space velocity of 3760 hr$^{-1}$ and at a pressure of 16 bars absolute. The performance of this catalys is shown in Table 8.

TABLE 8

| Chloride Level (ppm by volume) | R | S40 (%) | T40 (°C) | Test duration days | Catalyst Chloride Level (ppm) | | |
|---|---|---|---|---|---|---|---|
| | | | | | Front | Middle | Back |
| VC 0.8 ⎱ EC 0.8 ⎰ | 5000 | 80.5 | 214 | 7 | 106 | 150 | 145 |
| VC 2.5 ⎱ EC 2.5 ⎰ | 1600* | 79.5 | 233. | 13 | 1850 | 328 | 141 |

VC means vinyl chloride

EC means ethyl chloride

The second run is not comprised by claim 1.

Example 9

A catalyst was prepared as in Example 1. Rubidium was added as its nitrate using the method described in Example 1 from a solution containing 1.5 g/l rubidium (expressed as the element). This catalyst was tested as described in Example 5. 0.4 g of catalyst were loaded into a 2 mm diameter tube, length 10 cm. A gas mixture containing 30% ethylene, 8% oxygen, 4% carbon dioxide, 1.2 ppm vinyl chloride equivalents, 0.6% ethane and the remainder being nitrogen was passed over the catalyst at 16 bars pressure absolute, and the temperature adjusted to give an activity of 4 moles of ethylene converted/kg catalyst/hr and 40% conversion of oxygen. This required a temperature of 200°C at a selectivity of 75.5%.

The levels of cesium and rubidium in the catalysts in the above Examples are the amounts extractable by contact with dilute nitric acid.

In these Examples, parts per million (ppm) and percentages in the gas phase are by volume, and parts per million and percentages in catalysts are by weight. S40 is the selectivity expressed as moles of ethylene oxide produced per 100 moles of ethylene converted at an oxygen conversion of 40% and T40 is the temperature at which this occurred. Psig means pounds per square inch gauge. GHSV means gas hourly space velocity. R is the ratio of moles of ethane to moles of reaction inhibitor expressed as its vinyl chloride equivalent. Runs in which the value for R or the catalysts are marked ★ do not fall within our claims.

**Claims**

1. A process of producing ethylene oxide by contacting ethylene with oxygen optionally in the presence of diluent gases and in the presence of a chlorine containing reaction inhibitor and a silver containing catalyst which is promoted with potassium, rubidium and/or cesium characterised in that ethane is present in a ratio of at least 5,000 moles per mole of reaction inhibitor expressed as its vinyl chloride equivalent, the concentration of the reaction inhibitor expressed as its vinyl chloride equivalent is 0,2 to 10 parts per million by volume and that the potassium, rubidium and/or cesium content is at least $5 \times 10^{-6}$ gram atoms per gram of catalyst in a form which is extractable by contact with dilute nitric acid.

2. A process as claimed in Claim 1 in which at least 8,000 moles of ethane are present per mole of reaction inhibitor expressed as its vinyl chloride equivalent.

3. A process as claimed in Claim 1 or 2 in which more than $8 \times 10^{-6}$ gram atoms of potassium, rubidium and/or cesium are present per gram of catalyst in a form which is extractable by contact with dilute nitric acid.

4. A process as claimed in Claim 1, 2 or 3 in which at most 100,000 moles of ethane are present per mole of reaction inhibitor expressed as its vinyl chloride equivalent.

5. A process as claimed in any preceding claim in which the promoter is cesium.

12

6. A process as claimed in any preceding claim in which the reaction inhibitor is ethylene dichloride or vinyl chloride.

7. A process as claimed in any preceding claim in which the catalyst comprises 3 to 30% by weight of silver supported on a porous heat resisting support which has a specific surface area of 1 to 5 square meters per gram.

8. A process as claimed in any preceding claim in which the amount of ethane present is in the range 0.3 to 5% by volume of the gases fed to the reaction.

9. A process as claimed in any preceding claim in which the catalyst comprises silver supported on an alpha alumina support.

## Patentansprüche

1. Verfahren zur Herstellung von Ethylenoxid durch Inberührungbringen von Ethylen mit Sauerstoff, gegebenenfalls in Gegenwart von Verdünnungsgasen, in Gegenwart eines Chlor-enthaltenden Reaktionsinhibitors und eines Silber-enthaltenden Katalysators, der mit Kalium, Rubidium und/oder Cäsium aktiviert ist, dadurch gekennzeichnet, daß Ethan in einem Anteil von wenigstens 5000 mol pro Mol des Reaktionsinhibitors, ausgedrückt als sein Vinylchloridäquivalent, vorhanden ist, wobei die Konzentration des Reaktionsinhibitors, ausgedrückt als sein Vinylchloridäquivalent 0,2 bis 10 Volumenteile pro 1 Million Volumenteile beträgt, und daß das Kalium, Rubidium und/oder Cäsium in einer Menge von wenigstens $5 \times 10^{-6}$ Grammatome pro Gramm Katalysator in einer Form enthalten ist, die bei Kontakt mit verdünnter Salpetersäure extrahierbar ist.

2. Verfahren nach Anspruch 1, dadurch gekennzeichnet, daß wenigstens 8000 Mol Ethan pro Mol Reaktionsinhibitor, ausgedrückt als sein Vinylchloridäquivalent, vorhanden sind.

3. Verfahren nach Anspruch 1 oder 2, dadurch gekennzeichnet, daß mehr als $8 \times 10^{-6}$ Grammatome Kalium, Rubidium und/oder Cesium pro Gramm Katalysator in einer Form vorliegen, die bei Kontakt mit verdünnter Salpetersäure extrahierbar ist.

4. Verfahren nach einem der vorhergehenden Ansprüche, dadurch gekennzeichnet, daß höchstens 100.000 Mol Ethan pro Mol Reaktionsinhibitor, ausgedrückt als sein Vinylchloridäquivalent, vorhanden sind.

5. Verfahren nach einem der vorhergehenden Ansprüche, dadurch gekennzeichnet, daß der Beschleuniger Cäsium ist.

6. Verfahren nach einem der vorhergehenden Ansprüche, dadurch gekennzeichnet, daß der Reaktionsinhibitor Ethylendichlorid oder Vinylchlorid ist.

7. Verfahren nach einem der vorhergehenden Ansprüche, dadurch gekennzeichnet, daß der Katalysator 3 bis 30 Gew.-% Silber enthält, das auf einem porösen, hitzebeständigen Träger aufgebracht ist, welcher eine spezifische Oberfläche von 1 bis 5 m²/g hat.

8. Verfahren nach einem der vorhergehenden Ansprüche, dadurch gekennzeichnet, daß das Ethan in einer Menge im Bereich von 0,3 bis 5 Vol.-%, bezogen auf die der Reaktion zugeführten Gase, vorhanden ist.

9. Verfahren nach einem der vorhergehenden Ansprüche, dadurch gekennzeichnet, daß der Katalysator Silber enthält, das auf einen alpha-Aluminiumoxidträger aufgebracht ist.

## Revendications

1. Procédé de production d'oxyde d'éthylène par mise en contact d'éthylène avec de l'oxygène, éventuellement en présence de diluants gazeux et en présence d'un inhibiteur de réaction contenant du chlore, et avec un catalyseur contenant de l'argent qui contient comme promoteur du potassium, du rubidium et/ou du césium, caractérisé en ce que de l'éthane est présent dans un rapport d'au moins 5.000 moles par mole d'inhibiteur de réaction, exprimé sous la forme de son équivalent en chlorure de vinyle, la concentration de l'inhibiteur de réaction, exprimé sous la forme de son équivalent en chlorure de vinyle, est de 0,2 à 10 parties par million en volume et la teneur en potassium, rubidium et/ou césium est d'au moins $5 \times 10^{-6}$ atome-gramme par g de catalyseur sous une forme qui est extractible par contact avec de l'acide nitrique dilué.

2. Procédé suivant la revendication 1, dans lequel au moins 8.000 moles d'éthane sont présentes par mole d'inhibiteur de réaction, exprimé sous la forme de son équivalent en chlorure de vinyle.

3. Procédé suivant la revendication 1 ou 2, dans lequel plus de $8 \times 10^{-6}$ atome-gramme de potassium, de rubidium et/ou de césium sont présents par g de catalyseur sous une forme qui est extractible par contact avec de l'acide nitrique dilué.

4. Procédé suivant la revendication 1, 2 ou 3, dans lequel au maximum 100.000 moles d'éthane sont présentes par mole d'inhibiteur de réaction, exprimé sous la forme de son équivalent en chlorure de vinyle.

5. Procédé suivant l'une quelconque des revendications précédentes, dans lequel le promoteur est le césium.

6. Procédé suivant l'une quelconque des revendications précédentes, dans lequel l'inhibiteur de réaction est le dichlorure d'éthylène ou le chlorure de vinyle.

7. Procédé suivant l'une quelconque des revendications précédentes, dans lequel le catalyseur comprend 3 à 30% en poids d'argent déposé sur un support poreux résistant à la chaleur qui a une surface spécifique de 1 à 5 m$^2$/g.

8. Procédé suivant l'une quelconque des revendications précédentes, dans lequel la quantité d'éthane en présence est de 0,3 à 5% en volume des gaz admis à la réaction.

9. Procédé suivant l'une quelconque des revendications précédentes, dans lequel le catalyseur comprend de l'argent déposé sur un support d'alumine alpha.